# EUROPÄISCHE PATENTANMELDUNG

(11) **EP 4 745 114 A1**
(43) Veröffentlichungstag der Anmeldung: **20.05.2026**
(21) Anmeldenummer: 24213244.7
(22) Anmeldetag: 15.11.2024
(51) Int. Cl.: C07C 29/151, C07C 31/04

(54) **VERFAHREN ZUR METHANOLHERSTELLUNG**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: König, Sebastian, 60439 Frankfurt am Main (DE); Pena, Vincent, 60439 Frankfurt am Main (DE)
(74) Vertreter: Air Liquide

(57) **Zusammenfassung**

Die Erfindung betrifft eine Verfahren zur Herstellung von Methanol, bei dem ein erster Eduktstrom mit einem ersten Edukt in Form von Wasserstoff und ein zweiter Eduktstrom mit einem zweiten Edukt in Form eines kohlenstoffoxidhaltigen Gasgemisches bereitgestellt werden; das erste und das zweite Edukt in eine Synthesereaktoranordnung (3) eingespeist werden; und die Methanolsynthese in der Synthesereaktoranordnung (3) durchgeführt wird, wodurch ein Produktstrom, enthaltend Methanol, gebildet wird. Es werden Prognosen über Änderungsraten von Zuflussrate des ersten und/oder zweiten Edukts erstellt, um Schwankungen zu kompensieren.

## Beschreibung

### TECHNISCHES GEBIET

Die vorliegende Erfindung betrifft ein Verfahren und eine Anlage zur Herstellung von Methanol.

### HINTERGRUND DER ERFINDUNG

Das "Power-to-Methanol" Konzept integriert die Nutzung erneuerbarer Energiequellen durch die Umwandlung überschüssiger elektrischer Energie aus Wind- oder Solarkraftwerken in chemische Produkte. Der Prozess beginnt mit der Elektrolyse von Wasser, bei der elektrischer Strom durch einen Elektrolyseur geleitet wird. Dort wird Wasser in Sauerstoff und Wasserstoff gespalten. An der Anode findet die Oxidation statt, wobei Wasser zu Sauerstoff und Protonen oxidiert wird, während an der Kathode die Protonen zu Wasserstoffgas reduziert werden.

Der erzeugte Wasserstoff (H₂) bildet den Haupteduktstrom für die Methanolsynthese. Dieser Wasserstoff wird zusammen mit Kohlenmonoxid (CO) oder Kohlendioxid (CO₂) in einem speziellen Reaktor kombiniert, der mit einem Katalysator ausgestattet ist. Der Katalysator, oft eine Mischung aus Kupfer, Zinkoxid und Aluminiumoxid, fördert die chemische Reaktion, bei der Wasserstoff und CO/CO₂ zu Methanol umgewandelt werden. Nach der Synthese wird aus dem erhaltenen Produktgas ein Gemisch aus Methanol und Wasser, sogenanntes Rohmethanol, auskondensiert, und einer Destillation unterzogen, um nicht gewünschte Bestandteile wie Wasser und leichtflüchtige und/oder schwerflüchtige Nebenprodukte zu entfernen. Methanol hat eine niedrigere Siedetemperatur als Wasser, wodurch es durch Destillation gereinigt und aufkonzentriert werden kann.

Um gleichbleibende Produktionsraten bei der Methanolsynthese und Destillation zu gewährleisten, ist eine präzise Steuerung der Eduktströme und der Prozessbedingungen entscheidend. In der Methanolsynthese müssen die Zufuhr von Wasserstoff und CO/CO₂ sowie die Druck- und Temperaturbedingungen stabil geregelt werden, um eine effiziente Reaktionsrate und Katalysatoraktivität zu sichern.

Konventionelle Anlagen zur Synthese von Methanol sind dahingehend ausgelegt, dass sie kontinuierlich bei einer konstanten Auslastung produzieren. Änderungen in der Auslastung sind im Normalbetrieb minimal. Lastwechsel und Prozessanpassungen zwischen definierten, stationären Zuständen erfolgen sehr langsam über Stunden bis Tage, um die Methanolanlage und den Katalysator zu schützen. Schnelle Änderungen der Betriebsbedingungen können die Produktionsstabilität beeinträchtigen und unerwünschte Nebenprodukte verursachen. So kann eine plötzliche Änderung der Zufuhrmenge bzw. Zuflussrate von Wasserstoff oder CO/CO₂ die Reaktionsgeschwindigkeit stark beeinflussen, was zu einer unkontrollierten Erhöhung der Temperatur im Reaktor führen kann, und die Effizienz der Reaktion verringert. Dadurch wird die Qualität des erzeugten Methanols negativ beeinflusst. Auch kann es bei schnellen Änderungen der Zuflussraten zu einer ungleichmäßigen Verteilung der Gase kommen, was die Reaktionsrate und die Produktqualität negativ beeinflussen kann. Auch in der Destillation ist ein kontinuierlicher Betrieb essenziell, um eine gleichmäßige Produktqualität zu gewährleisten. Auch hier können schnelle Schwankungen in der Zuflussrate des Produktgases bzw. des Rohmethanols zu Instabilitäten im Destillationsprozess führen und die Trennungseffizienz beeinträchtigen. Daher sind stabile Zufuhrmengen und ein gut reguliertes Prozessmanagement entscheidend, um eine hohe Effizienz und Produktqualität in beiden Verfahren sicherzustellen.

Es ist Aufgabe der Erfindung, die Stabilität und Effizienz der Methanolherstellung zu verbessern.

### ZUSAMMENFASSUNG DER ERFINDUNG

Die der Erfindung zugrunde liegende Aufgabe wird gelöst mit einem Verfahren und einer Anlage gemäß den unabhängigen Ansprüchen. Weitere und bevorzugte Ausführungsformen der Erfindung sind in der nachfolgenden Beschreibung und in den abhängigen Ansprüchen offenbart.

Die Erfindung wird hinsichtlich mehrerer Aspekte beschrieben, die ein Verfahren und eine Anlage betreffen. Die Ausführungen zu den einzelnen Aspekten ergänzen einander, so dass die Ausführungen der Beschreibung des Verfahrens auch als Ausführungen der Beschreibung der Anlage, die zur Durchführung des Verfahrens eingerichtet ist, zu verstehen sind, und andersherum.

Erfindungsgemäß wird ein Verfahren zur Herstellung von Methanol vorgestellt, bei dem a) ein erster Eduktstrom mit einem ersten Edukt in Form von Wasserstoff und ein zweiter Eduktstrom mit einem zweiten Edukt in Form eines kohlenstoffoxidhaltigen Gases bereitgestellt werden; b) das erste und das zweite Edukt in eine Synthesereaktoranordnung eingespeist werden; und c) die Methanolsynthese in der Synthesereaktoranordnung durchgeführt wird, wodurch ein Produktstrom, enthaltend Methanol, gebildet wird. Das Verfahren sieht weiter folgende Schritte vor: Die Bereitstellung wenigstens eines ersten Pufferspeichers zur Zwischenspeicherung wenigstens eines der Edukte vor der Synthesereaktoranordnung; das Ermitteln der Zuflussrate des wenigstens einen Edukts in die Synthesereaktoranordnung; und das Erstellen einer Prognose hinsichtlich einer Änderungsrate der Zuflussrate auf der Grundlage von Einflussgrößen, von denen die Zuflussrate des wenigstens einen Edukts abhängig ist, wobei dann, wenn die Prognose eine positive Änderungsrate anzeigt, deren Betrag einen vordefinierten ersten Schwellenwert, vorzugsweise über einen vorbestimmten Zeitraum, überschreitet, zumindest ein Teil des Stroms des wenigstens einen Edukts in den ersten Pufferspeicher umgeleitet wird; und/oder wenn die Prognose eine negative Änderungsrate anzeigt, deren Betrag einen vordefinierten zweiten Schwellenwert, vorzugsweise über einen vorbestimmten Zeitraum, überschreitet, das wenigstens eine Edukt aus dem ersten Pufferspeicher in die Synthesereaktoranordnung eingespeist wird.

Indem eine Prognose über eine zukünftige Änderungsrate der Zuflussrate erstellt wird, kann der Methanol-Herstellungsprozess vorrausschauend geregelt werden. Durch die vorrausschauende Regelung der Zuflussrate des Stroms des wenigstens einen Edukts oder Eduktstroms wird die Geschwindigkeit mit der sich der Zufluss über Zeit ändert, verringert. Bildlich gesprochen wird anstatt eines abrupten Sprungs der eingebrachten Stoffmenge des betreffenden Edukts in die Synthesereaktoranordnung im zeitlichen Verlauf eine allmähliche Rampe erzeugt. Dies ermöglicht der Anlage einen schonenden Übergang zwischen Betriebszuständen.

Die Zuflussrate zeigt an, wie viel von einem bestimmten Stoff pro Zeiteinheit durch ein System fließt. Die Änderungsrate beschreibt, wie schnell sich diese Zuflussrate selbst ändert. Wenn die Änderungsrate positiv ist, also eine positive Steigung hat, nimmt die Zuflussrate zu. Ist die Änderungsrate negativ, also hat sie eine negative Steigung, nimmt die Zuflussrate ab. Der Betrag der Änderungsrate gibt an, wie schnell diese Veränderung geschieht.

Schwellenwerte sind bestimmte Werte, die festgelegt werden, um zu überwachen, ob die Änderungsrate der Zuflussrate zu schnell steigt oder sinkt. Der erste Schwellenwert zeigt einen zu schnellen Anstieg der Zuflussrate an. Der zweite Schwellenwert zeigt eine zu schnelle Abnahme der Zuflussrate an. Die Schwellenwerte definieren zwischen sich einen Bereich, innerhalb dessen die Änderungsrate der Zuflussrate als akzeptabel angesehen wird. Die Entscheidung über die weiteren Maßnahmen auf Grundlage des ersten oder des zweiten Schwellenwerts kann auf Grundlage der Steigung (Ableitung) der Änderungsrate getroffen werden. Dem ersten Schwellenwert ist eine positive Steigung der Änderungsrate zugeordnet, weil diese eine Zunahme der Zuflussrate anzeigt, d.h., zumindest ein Teil des Stroms des wenigstens einen Edukts wird dann in den ersten Pufferspeicher umgeleitet, wenn die Prognose eine Änderungsrate mit positiver Steigung oder positivem Vorzeichen anzeigt, deren Betrag einen vordefinierten ersten Schwellenwert überschreitet. Dem zweiten Schwellenwert ist eine negative Steigung der Änderungsrate zugeordnet, weil diese eine Abnahme der Zuflussrate anzeigt, d.h., das wenigstens eine Edukt aus dem ersten Pufferspeicher wird in die Synthesereaktoranordnung eingespeist, wenn die Prognose eine Änderungsrate mit negativer Steigung oder negativem Vorzeichen anzeigt, deren Betrag einen vordefinierten zweiten Schwellenwert überschreitet.

Grundsätzlich kann vorgesehen sein, den oder die Pufferspeicher zumindest teilweise zu füllen, um auf Änderungen der Zuflussrate vorbereitet zu sein.

Das erste und das zweite Edukt werden in Eduktströmen bereitgestellt. Das erste Edukt und das zweite Edukt können im Einlass der Synthesereaktoranordnung und/oder vor dem Einlass der Synthesereaktoranordnung gemischt werden, wodurch ein Feedgasstrom erhalten wird. Die Zuflussrate kann sich auf das Edukt an sich, also z.B. H₂, oder auf einen Eduktstrom, der das Edukt enthält, beziehen. Das zweite Edukt in Form des kohlenstoffoxidhaltigen Gases kann ein Gasgemisch sein, z.B. ein Gas, das eine kohlenstoffoxidhaltige Komponente aufweist. Das zweite Edukt kann auch ein reiner Kohlendioxidstrom sein.

Im Rahmen des erfindungsgemäßen Verfahrens und der Anlage umfasst die Zwischenspeicherung des ersten und/oder zweiten Edukts mittels erster Pufferspeicher wenigstens folgende Fälle:
1.) Ein erster Eduktstrom mit dem ersten Edukt wird bereitstellt, der in dem ersten Pufferspeicher zwischengespeichert werden kann. Ein zweiter Eduktstrom mit dem zweiten Edukt ist nicht an den ersten Pufferspeicher angeschlossen.
2.) Ein zweiter Eduktstrom mit dem zweiten Edukt wird bereitstellt, der in dem ersten Pufferspeicher zwischengespeichert werden kann. Ein erster Eduktstrom mit dem ersten Edukt ist nicht an den ersten Pufferspeicher angeschlossen.
3.) Ein Feedgasstrom enthaltend das erste und das zweite Edukt wird bereitgestellt, der in dem ersten Pufferspeicher zwischengespeichert werden kann.
4.) Ein erster Eduktstrom mit dem ersten Edukt und ein zweiter Eduktstrom mit dem zweiten Edukt können in separaten ersten Pufferspeichern zwischengespeichert werden.

Die Prognose oder Vorhersage der Änderungsrate des Zuflusses eines oder mehrerer Eduktströme in die Synthesereaktoranordnung kann mithilfe datenbasierter Vorhersagemodelle erstellt werden. Diese Modelle nutzen mathematische Darstellungen des Methanolherstellungsprozesses, um das Systemverhalten zu beschreiben und Simulationen durchzuführen. Auf Grundlage historischer Produktionsdaten, Verbrauchsmuster und anderer Einflussgrößen auf die Eduktbereitstellung können Vorhersagen zur Bereitstellung von H₂ und CO₂/CO getroffen werden. So kann vorhergesagt werden, dass eine Reduktion der Energieversorgung um X% für die H₂-Elektrolyse die Wasserstoffproduktion um Y% verringert. Das Vorhersagemodell wird mit Trainingsdaten trainiert, die den Einflussgrößen der Eduktbereitstellung zugeordnet sind.

Eine Steuereinrichtung, die das Vorhersagemodell verwendet, überwacht die Methanolproduktion und berechnet die Wahrscheinlichkeit, mit der eine Änderung des Zuflusses bzw. der Zuflussrate der Eduktströme auftritt. Zusätzlich wird die Wahrscheinlichkeit berechnet mit welcher Änderungsrate die Veränderung des Zuflusses auftritt. Das Modell kann Messdaten eines Satzes von Sensoren, z.B. zur Messung der Gasströme, in der Methanolanlage zur Berechnung dieser Wahrscheinlichkeit nutzen. Wenn die berechnete Wahrscheinlichkeit einen bestimmten Schwellenwert überschreitet, gilt das Auftreten einer Änderung der Zuflussrate bzw. das Auftreten einer Änderungsrate als prognostiziert. Zeigt die Prognose eine Änderungsrate an, die vordefinierte Schwellenwerte über bzw. unterschreitet, führt die Steuereinrichtung eine zugeordnete Aktion in der Anlage aus.

Eine weitere Ausführungsform sieht daher vor, dass die Prognose bzw. Vorhersage das Auswerten von Einflussgrößen umfasst, insbesondere von Leistungsdaten von Betriebsparametern des Methanol-Herstellungsprozesses, von denen die Zuflussrate des wenigstens einen Edukts abhängig ist bzw. diese determiniert; und das Berechnen einer Wahrscheinlichkeit für das Auftreten einer Änderungsrate, wobei die Änderungsrate als prognostiziert gilt, wenn die Wahrscheinlichkeit einen vorbestimmten Schwellenwert überschreitet.

Einflussgrößen, wie Betriebsparameter der Methanol-Herstellung, die vom Vorhersagemodell zum Erstellen von Prognosen herangezogen werden können, umfassen unter anderem:
- Zuflussrate des Edukts oder Eduktstroms und damit verbundene Einflussgrößen, wie:
   - die Bereitstellung von Wasserstoff, wie z.B. Leistungsdaten eines Wasserelektrolyseurs (wie z.B. Wasserstoff-Ausstoßrate oder/oder der Energieverbrauch des Elektrolyseurs); Verfügbarkeit von Energie - insbesondere regenerativer Energie - zur Wasserelektrolyse, einschließlich Wetterbedingungen, Elektrolyt-Verfügbarkeit und Volumenstrom
   - die Bereitstellung des kohlenstoffoxidhaltigen Gases, wie z.B. Leistungsdaten einer CO₂ Abscheideanlage oder einer CO- und/oder Synthesegas-Produktionsanlage (wie z.B. CO/CO₂ Ausstoßrate, Druckverhältnisse, Temperatur und/oder Füllstand)
- Betriebsparameter der ersten Pufferspeicher, wie Füllstand, Temperatur, und/oder Druck.
- Betriebsparameter der Synthesereaktoranordnung, wie z.B. Druck, Temperatur, Ausstoßrate des Produktstroms und/oder Energieverbrauch; Zuflussrate der Edukte bzw. Eduktströme
- Betriebsparameter einer der Synthesereaktoranordnung nachgeschalteten Destillation wie z.B. Druckverhältnisse, insbesondere Druckabfall über einem Katalysator, Temperatur und/oder Energieverbrauch
- Betriebsparameter eines zweiten Pufferspeichers, der zwischen Synthesereaktoranordnung und Destillation angeordnet ist, wie Füllstand, Temperatur, und/oder Druck.

Die Messungen der obigen Einflussgrößen kann z.B. mittels Sensoren erfolgen, die in einer Anlage zur Herstellung von Methanol verteilt sind. Die Nennung der obigen Einflussgrößen ist nicht abschließend. Weitere und/oder andere Parameter, die für eine Prognose der Änderungsrate herangezogen werden können, lassen sich aus der rechnerischen Modellierung des zugrundeliegenden Systems zur Methanol-Herstellung ableiten.

Um die Änderungsrate der Zuflussrate eines Gasstroms in die Synthesereaktoranordnung auf einen vordefinierten Wert oder vordefinierten Bereich einzustellen, kann ein Regelsystem vorgesehen sein, z.B. ein Regelsystem, das als Teil einer Steuereinrichtung auf einem Datenverarbeitungsgerät mit Hardware-Prozessor implementiert ist. Dieses ist bevorzugt zur Durchführung folgender Schritte eingerichtet:
- Messung des Istwerts der Zuflussrate des wenigstens einen Edukts oder des Eduktstroms.
- Erzeugen eines Sollwerts für Zufuhr des Edukts oder Eduktstroms in die Synthesereaktoranordnung. Der Sollwert kann auf Grundlage der Konstruktion der Synthesereaktoranordnung bestimmt werden, beispielsweise durch die Nutzung eines Modells, das die Betriebsbedingungen und die Leistungsanforderungen der Anlage berücksichtigt.
- Vergleich des Ist-Werts mit dem vorgegebenen Sollwert.
- Bestimmen und Anpassen einer Steuervariable, die die Zuflussrate beeinflusst, basierend auf der festgestellten Abweichung zwischen Sollwert und Istwert.

Als Steuervariablen kommen unter anderem in Betracht:
- das Umlenken der Gasströme, z.B. des wenigstens einen Edukts in einen ersten Puffertank oder durch das Auslassen des wenigstens einen Edukts aus dem ersten Puffertank, z.B. mittels Ventile und Pumpen, elektrischen Stellglieder, elektrischen Schaltungen, etc., das Lenken von Wärmeströmen und elektrische Energie

Das Regelungssystem arbeitet kontinuierlich in einer Rückkopplungsschleife, indem es den Istwert des Gasstroms misst, mit dem Sollwert des Gasstroms vergleicht und die Steuervariablen entsprechend anpasst. Auf diese Weise wird die Zuflussrate des betreffenden Eduktstroms aktiv geregelt und die Änderungsrate in den gewünschten Bereich gesteuert, um eine stabile und zuverlässige Prozessführung sicherzustellen.

Die Zwischenspeicherung wenigstens eines der Edukte im ersten Pufferspeicher ist bevorzugt derart, dass das erste Edukt in Form von Wasserstoff gasförmig gespeichert wird. Das zweite Edukt in Form des kohlenstoffoxidhaltigen Gases wird bevorzugt flüssig gespeichert.

Nach einer weiteren Ausführungsform der Erfindung ist vorgesehen, dass das erste und das zweite Edukt in getrennten Eduktströmen bereitgestellt werden, wobei wenigstens für einen der Eduktströme ein separater Pufferspeicher bereitgestellt wird.

Nach einer weiteren Ausführungsform der Erfindung ist vorgesehen, dass beide Eduktströme in separaten ersten Pufferspeichen zwischengespeichert werden.

Nach einer weiteren Ausführungsform der Erfindung ist vorgesehen, dass der in der Synthesereaktoranordnung produzierte Produktstrom in eine Destillation geleitet wird. Außerdem ist vorgesehen, dass ein zweiter Pufferspeicher zur Zwischenspeicherung des Produktstroms bereitgestellt wird und dass die Zuflussrate des Produktstroms in die Destillation ermittelt wird. Ferner ist vorgesehen, dass der Produktstrom zumindest teilweise in den zweiten Pufferspeicher umgeleitet wird oder ein Produktstrom aus dem zweiten Pufferspeicher in die Destillation eingespeist wird, um Änderungen der Zuflussrate des Produktstroms von der Synthesereaktoranordnung auszugleichen. Der weitere Pufferspeicher ermöglicht eine stabilere Zufuhr des Produktstroms zur Destillation. Da die Zuflussrate aus der Synthesereaktoranordnung variieren kann, dient der Pufferspeicher als Zwischenspeicher, der Schwankungen des Produktstroms abfedert. Dadurch wird die Destillation mit einem gleichmäßigeren Zulauf versorgt, was die Effizienz der Trennprozesse erhöht.

Nach einer weiteren Ausführungsform der Erfindung ist vorgesehen, dass eine Prognose hinsichtlich einer Änderungsrate der Zuflussrate des Produktstroms in die Destillation auf der Grundlage von Einflussgrößen, von denen die Zuflussrate des Produktstroms abhängig ist, erstellt wird. Wenn die Prognose eine positive Änderungsrate anzeigt bzw. vorhersagt, deren Betrag einen vordefinierten dritten Schwellenwert überschreitet, wird zumindest ein Teil des Produktstroms in den zweiten Pufferspeicher umgeleitet; und/oder wenn die Prognose eine negative Änderungsrate anzeigt bzw. vorhersagt, deren Betrag einen vordefinierten vierten Schwellenwert überschreitet, wird ein Produktstrom aus dem Pufferspeicher in die Destillation eingespeist, um die Änderungsrate der Zuflussrate des Produktstroms in die Destillation auf einen vordefinierten Wert oder vordefinierten Bereich einzustellen. Die Erstellung einer Vorhersage über den Produktstrom bzw. die Produktstrom-Zuflussrate kann auf den gleichen Grundlagen erfolgen, wie es in Bezug auf den oder die Eduktströme erläutert wurde. Als Einflussgrößen und Betriebsparameter, die zum Erstellen der Prognose herangezogen werden können, gehören die bereits in Bezug auf die Eduktströme erläuterten Größen.

Nach einer weiteren Ausführungsform der Erfindung ist vorgesehen, dass das zweite Edukt Kohlenstoffdioxid und/oder Kohlenstoffmonoxid und/oder ein Synthesegasgemisch, welches zumindest Wasserstoff und Kohlenmonoxid umfasst, ist.

Vorzugsweise wird der Wasserstoff durch Wasserelektrolyse, vorzugsweise zumindest teilweise mit erneuerbarer Energie, bereitgestellt. Vorzugsweise wird ein Elektrolyseur verwendet, der mit der Synthesereaktoranordnung fluidverbunden ist, d.h., der Elektrolyseur ist in die Anlage, aufweisend die Synthesereaktoranordnung, eingebunden.

Nach einer weiteren Ausführungsform der Erfindung ist vorgesehen, dass die Erstellung einer Prognose über die Zuflussrate des ersten Edukts eine Vorhersage hinsichtlich der Energiebereitstellung für die Wasserelektrolyse beinhaltet. Dies kann insbesondere auf Grundlage von technischen oder marktwirtschaftlichen Einflussgrößen, die die Bereitstellung von Energie für die Wasserelektrolyse beeinflussen oder bestimmen, erfolgten. Zukünftige Änderungen in der Verfügbarkeit von Energie für die Elektrolyse können auf verschiedene Weisen abgeschätzt oder ermittelt werden. Eine Möglichkeit ist die Analyse historischer Daten, um vergangene Energieverbrauchsmuster und -trends für ähnliche Prozesse oder Anlagen zu verstehen. Zudem spielen Marktanalysen eine Rolle, indem sie Faktoren wie Energiepreise und die Verfügbarkeit von Energiequellen berücksichtigen, die die zukünftige Verfügbarkeit beeinflussen können. Schließlich ermöglichen Simulationen die Modellierung verschiedener Szenarien für die Energiebereitstellung, z.B., indem der Energieaufwand und die Energieverfügbarkeit einer gesamten Anlage, z.B., eines Werks, simuliert und unter verschiedenen Betriebsbedingungen ausgewertet werden. Auf diesen Grundlagen lässt sich eine Vorhersage über die Energiebereitstellung für die Elektrolyse aufstellen, was wiederum die Vorhersage der Zuflussrate des ersten Edukts ermöglicht. Dies ist insbesondere hinsichtlich der regenerativen Energien von Vorteil, da deren Verfügbarkeit oft Schwankungen unterliegen.

Nach einer weiteren Ausführungsform der Erfindung ist vorgesehen, dass das erste Edukt durch Wasserelektrolyse bereitgestellt wird und das Erstellen einer Prognose hinsichtlich der Energiebereitstellung für die Wasserelektrolyse auf Grundlage von Parametern, die die Bereitstellung von Energie für die Wasserelektrolyse beeinflussen bzw. bestimmen. Außerdem ist ein Ramp-up-Betrieb vorgesehen, bei dem ein prognostizierter Anstieg einer Energiebereitstellung für die Wasserelektrolyse zu einer Erhöhung der Zuflussrate des ersten Edukts führt, wobei das erste Edukt zumindest teilweise in dem ersten Pufferspeicher derart zwischengespeichert wird, dass die Änderungsrate der Zuflussrate des in die Synthesereaktoranordnung eingespeisten ersten Edukts auf eine vordefinierte Rate begrenzt wird. Somit kann optimal auf einen vorhergesagten Anstieg der Wasserstoffproduktion infolge zunehmender Energieverfügbarkeit reagiert werden. Insbesondere kann die Zufuhr von Wasserstoff auf eine für die Methanolsynthese akzeptable oder optimale Rate begrenzt werden. Nach einer weiteren Ausführungsform der Erfindung ist alternativ oder zusätzlich ein Ramp-down-Betrieb, vorgesehen, bei dem ein prognostizierter Rückgang einer Energieversorgung für die Wasserelektrolyse zu einer Verringerung der Zuflussrate des ersten Edukts führt, wobei das erste Edukt aus dem ersten Pufferspeicher in die Synthesereaktoranordnung derart eingespeist wird, dass die Änderungsrate der Zuflussrate des in die Synthesereaktoranordnung eingespeisten ersten Edukts auf eine vorbestimmte Rate begrenzt wird. Damit kann optimal auf eine vorhergesagte Reduzierung der Wasserstoffproduktion infolge abnehmender Energieverfügbarkeit reagiert werden. Während die Methanolproduktion in der Methanolsynthese abnimmt, erfüllt der zweite Pufferspeicher eine Pufferfunktion, um die Methanolzufuhr auf eine für die Methanoldestillation effektive Menge zu begrenzen.

Nach einer weiteren Ausführungsform der Erfindung ist vorgesehen, dass Wärme aus der Synthesereaktoranordnung und/oder der Destillation entnommen und zwischengespeichert und/oder durch Energiezufuhr erzeugt wird, wobei das Verfahren ferner einen Standby-Betrieb umfasst, wobei der Standby -Betrieb aufgenommen wird, wenn die prognostizierte Zuflussrate wenigstens eines der ersten und zweiten Edukte in die Synthesereaktoranordnung, vorzugsweise über einen vorbestimmten Zeitraum, unter einen vorbestimmten Schwellenwert sinkt, wobei Druck- und Temperaturbedingungen in der Synthesereaktoranordnung auf vorbestimmte Werte geregelt werden, wobei die durch Energiezufuhr erzeugte Wärme in die Synthesereaktoranordnung und/oder die Destillation geleitet wird. Zum Aufrechterhalten der Druck- und Temperaturverhältnisse wird Wärme in die Synthesereaktoranordnung eingeleitet. Dadurch ist eine schnelle Aufnahme des regulären Betriebes nach Aufheben des Stand-by-Betriebs möglich. Vorzugsweise werden im Stand-by-Betrieb die Zuflüsse des ersten und/oder zweiten Edukts in die Synthesereaktoranordnung unterbrochen. Der Stand-by-Betrieb ist energieeffizient und ermöglicht eine flexible Betriebsstrategie.

Nach einer weiteren Ausführungsform der Erfindung ist vorgesehen, dass der Standby-Betrieb wieder aufgehoben wird, wenn eine prognostizierte Zuflussrate von wenigstens einem der Eduktströme einen vorbestimmten Schwellenwert, vorzugsweise über einen vorbestimmten Zeitraum erreicht, wobei dann die Einleitung der Wärme in die Synthesereaktoranordnung und/oder die Destillation unterbrochen werden kann. Außerdem wird die Einspeisung von erstem und/oder zweitem Edukt wieder aufgenommen.

Ein weitere Aspekt der Erfindung betrifft eine Anlage zur Methanolherstellung, umfassend eine Synthesereaktoranordnung, eine Destillation; eine oder mehrere Quellen für ein erstes Eduktgas und ein zweites Eduktgas, wobei die eine oder die mehreren Quellen über eine gemeinsame oder separate erste Leitungen mit der Synthesereaktoranordnung verbunden sind, wobei wenigstens eine der Leitungen mit einem ersten Pufferspeicher verbunden ist; und eine zweite Leitung (Rohmethanolleitung), die die Synthesereaktoranordnung mit der Destillation verbindet, wobei die zweite Leitung mit einem zweiten Pufferspeicher verbunden ist; und eine Steuereinrichtung, die dazu eingerichtet ist, die Anlage mit einem der hierin beschriebenen Verfahren zu betreiben.

Die Steuereinrichtung kann als Software auf einem Datenverarbeitungsgerät mit einem Hardware-Prozessor ablaufen. Dieses kann die hierein beschriebenen Regelsysteme und Prognosefunktionen in Form von Software aufweisen. Die Software kann Signale von in der Anlage vorgesehenen Sensoren empfangen und auswerten, sowie weitere Daten, wie z.B. die Energieverfügbarkeit aus dem Stromnetz, die als Betriebsparameter und Einflussgrößen für die Erstellung der Prognosen und die Steuerung und Regelung der Anlage verarbeitet werden. Außerdem kann die Steuereinrichtung mit Stellglieder oder Steuergliedern der Anlage wirkverbunden sein, um diese zu steuern. Zu den Stellgliedern können unter anderem Ventile, Pumpen, elektrische Schalter, Energieversorgung und weitere Steuerelemente gehören, die zur Steuerung und Regelung, insbesondere der Gasströme, Wärmeeinspeisung, etc., einer Anlage durch die Steuereinrichtung verwendet werden können.

### BESCHREIBUNG DER FIGUREN

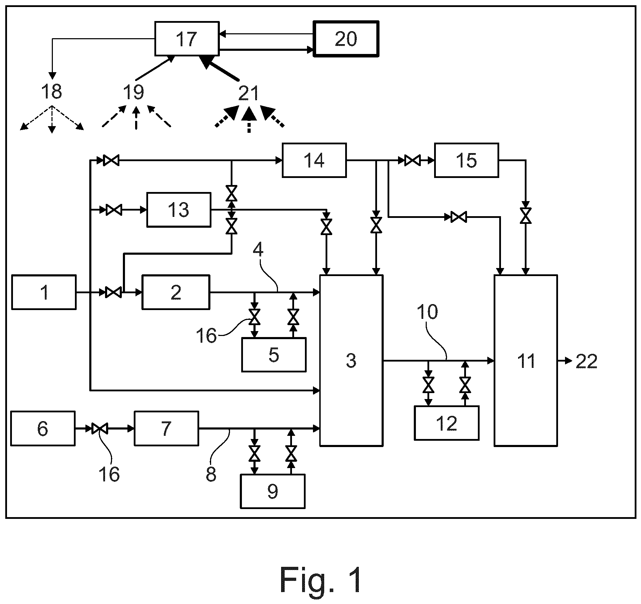

Die Erfindung wird nachfolgend anhand der FIG. 1 näher erläutert.
- FIG.1: zeigt eine schematische Darstellung einer ersten Ausführungsform einer erfindungsgemäßen Anlage zur Methanolherstellung.

Die Anlage umfasst eine Energieversorgung 1, einen Elektrolyseur 2 zur Wasserstoffelektrolyse, d.h. zur Erzeugung eines ersten Edukts in Form von Wasserstoff (H₂). Der Elektrolyseur 2 wird über die Energieversorgung 1 mit Strom versorgt, was die Verbindung zwischen der Energieversorgung 1 und dem Elektrolyseur 2 anzeigt.

Der Elektrolyseur 2 ist mit einer Synthesereaktoranordnung 3 zur Methanolsynthese über eine erste Leitung 4 strömungsverbunden. An die erste Leitung 4 ist ein erster Pufferspeicher 5 angeschlossen.

Eine CO₂ Quelle 6 ist strömungsverbunden mit einer CO₂-Abscheideeinheit 7, die wiederum über eine zweite Leitung 8 mit der Synthesereaktoranordnung 3 strömungsverbunden ist. An die zweite Leitung 8 ist ein weiterer erster Pufferspeicher 9 für CO₂ angeschlossen.

Die Synthesereaktoranordnung 3 ist über eine Rohmethanolleitung 10 mit einer Methanoldestillation 11 verbunden. An die Rohmethanolleitung 10 ist ein zweiter Pufferspeicher 12 angeschlossen.

Die Anlage umfasst weiter einen Energiespeicher 13 zur Speicherung elektrischer Energie, einen Strom-zu-Wärme-Umwandler 14 und einen Speicher für thermische Energie 15. Der Energiespeicher 13 und der Strom-zu-Wärme-Umwandler 14 sind mit der Energieversorgung 1 elektrisch verbunden sind. Der Speicher für thermische Energie 15 ist mit dem Strom-zu-Wärme-Umwandler 14 thermisch verbunden.

Die gezeigten Verbindungen zwischen den oben beschriebenen Komponenten stellen Wirkverbindungen dar. Zwischen elektrischen Komponenten sind dies insbesondere elektrische Leitungen, zwischen Komponenten, die strömungsverbunden sind, sind dies insbesondere Gas- bzw. Fluidleitungen. Die in den Verbindungen dargestellten Zeichen 16 stellen Stellglieder dar, wie Schaltelemente zwischen elektrischen Komponenten, oder Ventile zwischen Komponenten, die strömungsverbunden sind. Durch die Stellglieder 16 zwischen dem ersten Pufferspeicher 5 und der ersten Leitung 4 kann z.B. der Zufluss von Wasserstoff aus dem Elektrolyseur 2 in den ersten Pufferspeicher 5 und aus diesem heraus in die erste Leitung 4 gesteuert werden.

Eine Steuereinrichtung 17 läuft auf einem Datenverarbeitungsgerät (nicht dargestellt). Diese kann Steuerbefehle 18, z.B. drahtlos, an die Stellglieder 16 senden. Außerdem empfängt die Steuereinrichtung 17 über Sensoren (nicht dargestellt), die in der Anlage, insbesondere in den gezeigten Komponenten (Elektrolyseur, Synthesereaktoranordnung, Destillation, etc.) und den Leitungen, verteilt sind, Leistungsdaten 19 von Betriebsparametern, wie z.B. der Zuflussrate von Wasserstoff vom Elektrolyseur 2 in die Synthesereaktoranordnung 3, in Echtzeit.

Die Steuereinrichtung 17 ist mit einem ebenfalls auf dem Datenverarbeitungsgerät laufenden Simulations-/Vorhersagemodell 20 in Datenaustausch. Das Simulations-/Vorhersagemodell 20 ist eine mathematische Repräsentation der Anlage und empfängt Echtzeitdaten 19 von der Anlage und verwendet diese Daten, um das Verhalten der Anlage in Echtzeit nachzubilden sowie mittels Vorhersagemodellen Änderungen von Betriebszuständen vorherzusagen. Das Simulations-/Vorhersagemodell 20 kann basierend auf den aktuellen Betriebsbedingungen und weiteren Parametern die Verfügbarkeit elektrischer Energie in einer nächsten Zeitperiode vorhersagen und wieviel Wasserstoff in der nächsten Zeitperiode produziert werden wird. Diese Prognosedaten 21 fließen in die Steuereinrichtung 17 ein.

Im regulären Betriebsmodus stellt die Energieversorgung 1 Energie für den 2 Elektrolyseur 2 zur Verfügung. Dieser erzeugt ein erstes Edukt in Form von Wasserstoff. Dieser gelangt als erster Eduktstrom über die erste Leitung 4 in die Synthesereaktoranordnung 3. Außerdem wird über die zweite Leitung 8 ein zweiter Eduktstrom mit einem zweiten Edukt in Form von CO₂ in die Synthesereaktoranordnung 3 eingespeist. Dort findet die Methanolsynthese gemäß der Reaktion CO₂ + 3H₂ → CH₃OH + H₂O statt. Es entsteht ein Produktstrom enthaltend Rohmethanol, der über die Rohmethanolleitung 10 in die Methanoldestillation 11 gespeist wird. Nach der Destillation wird reines Methanol 22 ausgegeben.

Im Folgenden werden beispielhaft drei Betriebsmodi vorgestellt.

### Ramp-up-Betrieb:

In einem Ramp-up-Betrieb wird ein zu schneller Anstieg der Zuflussrate von CO₂ und/oder H₂ in die Synthesereaktoranordnung 3 kompensiert. Beispielhaft wird ein Ramp-up-Betrieb beschrieben, bei dem ein Anstieg der Energieversorgung für den Elektrolyseur 2 vorhergesagt wird. Das Simulationsmodell 20 erhält Echtzeitdaten über Betriebsparameter von den Komponenten der Anlage. Auf Grundlage von u.a. Modellberechnungen und historischen Daten errechnet die Steuereinrichtung 17 mit Hilfe des Simulations-/Vorhersagemodell 20 eine Prognose über die bereitgestellte Energie für eine zukünftige Zeitperiode. Eine prognostizierte Erhöhung der Energiezufuhr würde zu einer drastischen Erhöhung der Wasserstoff-Produktion des Elektrolyseurs 2 und des Wasserstoff-Zuflusses in die Synthesereaktoranordnung 3 führen. Die Steuereinrichtung 17, in Zusammenarbeit mit dem Simulations-/Vorhersagemodell 20, berechnet daraufhin die Geschwindigkeit, mit der sich die Zuflussrate von Wasserstoff ändern wird. Diese Geschwindigkeit wird durch die Änderungsrate der Zuflussrate ausgedrückt.

Die Steuereinrichtung 17 vergleicht die Steigung (positiv oder negativ) der prognostiziere Änderungsrate der Zuflussrate des Wasserstoffs und ihren Betrag mit gespeicherten Änderungsrate-Werten. Wenn festgestellt wird, dass der Betrag einer prognostizieren Änderungsrate mit positivem Vorzeichen einen vordefinierten Schwellenwert für einen bestimmten Zeitraum überschreitet, beginnt die Steuereinrichtung 17 bereits vor dem Eintritt des Energieanstiegs mit Kompensationsmaßnahmen und leitet einen Teil des ersten Eduktstroms vom Elektrolyseur 2 in der ersten Leitung 4 in den ersten Pufferspeicher 5 um, indem die entsprechenden Ventile zwischen erster Leitung 4 und erstem Pufferspeicher 5 angesteuert werden. Dadurch wird die Geschwindigkeit, mit der sich die Zuflussrate des Eduktstroms ändert, gesenkt. Der erste Pufferspeicher 5 dient dabei als Puffer. Durch die Umleitung des Wasserstoffs wird die Zuflussrate des Wasserstoffs zudem auf eine auf für die Methanolsynthese effektive maximale Zuflussrate begrenzt.

Mit steigender Zuflussrate des Wasserstoffs steigt auch die Menge an Produktgas und damit an kondensiertem Rohmethanol, das aus der Synthesereaktoranordnung 3 ausgegeben und zur Destillation 11 geleitet wird. Um die Zuflussrate von Rohmethanol in die Destillation 11, aber auch die Änderungsrate der Zuflussrate von Rohmethanol auf eine für die Destillation effektive Maximalrate zu begrenzen, steuert die Steuereinrichtung 17 Ventile in der Rohmethanolleitung 10 an, um einen Teil des Rohmethanols in den zweiten Pufferspeicher 12 umzulenken.

Wenn die Methanolproduktion in der Synthesereaktoranordnung 3 die Verarbeitungskapazität der Destillation 11 erreicht, wird der Überschuss an Rohmethanol im zweiten Pufferspeicher 12 aufgenommen.

### Ramp-down-Betrieb:

In einem Ramp-down-Betrieb wird ein zu schnelles Abfallen der Zuflussrate von CO₂ und/oder H₂ in die Synthesereaktoranordnung 3 kompensiert. Ein schnelles Abfallen der Zuflussrate kann z.B. durch einen drastischen Abfall der Energieversorgung verursacht werden. Wenn die Steuereinrichtung 17 einen Abfall der Energieversorgung und damit verbunden einen Abfall der Wasserstoffproduktion und der Zuflussrate von Wasserstoff prognostiziert, wird die ebenfalls prognostiziere Änderungsrate der Zuflussrate mit einem vordefinierten Schwellenwert verglichen. Liegt der Betrag einer prognostizierten Änderungsrate der Zuflussrate mit negativem Vorzeichen für einen bestimmten Zeitraum oberhalb eines vordefinierten Schwellenwerts, wird über die Ansteuerung von Ventilen zwischen dem ersten Pufferspeicher 5 und der ersten Leitung 4 Wasserstoff, der in dem ersten Pufferspeicher 5 zwischengespeichert ist, in die erste Leitung 4 und die Synthesereaktoranordnung 3 eingespeist. Der erste Pufferspeicher 5 kompensiert die Geschwindigkeit der Reduzierung der Wasserstoffversorgung in die Synthesereaktoranordnung 3 auf ein für die Methanolsynthese effektives Maß. Außerdem wird der erste Pufferspeicher 5 genutzt, um die Zuflussrate des Wasserstoffs auf einer auf für die Methanolsynthese effektiven Zuflussrate zu halten, sollte die Wasserstoffproduktion weiterabfallen.

Mit sinkender Zuflussrate des Wasserstoffs sinkt auch die Menge an Rohmethanol, das aus der Synthesereaktoranordnung 3 ausgegeben und zur Destillation 11 geleitet wird. Um die Zuflussrate von Rohmethanol in die Destillation, aber auch die Änderungsrate der Zuflussrate von Rohmethanol auf für die Destillation effektive Raten zu begrenzen, steuert die Steuereinrichtung 17 Ventile in der Rohmethanolleitung 10 an, um Rohmethanol aus dem zweiten Pufferspeicher 12 in die Destillation 11 zu speisen.

Wenn die Methanolproduktion in der Synthesereaktoranordnung 3 unterhalb eine Mindestverarbeitungskapazitätsgrenze der Destillation 11 liegt, wird der Betrieb der Destillation zunächst durch Einspeisung von Rohmethanol aus dem zweiten Pufferspeicher 12 aufrechterhalten. Wenn kein Rohmethanol mehr zur Verfügung gestellt werden kann, kann die Destillation 11 in einen Rückflussmodus versetzt werden, in dem Destillationsprodukte intern zirkuliert werden.

### Stand-by-Betrieb:

Ferner ist die Steuereinrichtung 17 für einen Standby-Betrieb ausgelegt. Wenn die Steuereinrichtung 17 prognostiziert, dass die Wasserstoffproduktion für eine längeren Zeitraum unzureichend ist oder nicht stattfinden kann, kann die Anlage in einen Ruhemodus versetzt werden. In diesem Modus werden Druck- und Temperaturverhältnisse in der Synthesereaktoranordnung 3 auf einem definierten Niveau eingestellt, um ein schnelles Anfahren der Synthesereaktoranordnung 3 nach Beendigung des Standby-Betriebs zu gewährleisten. Zu diesem Zwecke wird über den Strom-zu-Wärme-Umwandler 14 und den Speicher für thermische Energie 15 Wärme in die Synthesereaktoranordnung 3 und die Destillation eingebracht.

### Bezugszeichenliste

- 1: Energieversorgung
- 2: Elektrolyseur
- 3: Synthesereaktoranordnung
- 4: Erste Leitung
- 5: Erster Pufferspeicher
- 6: CO2 Quelle
- 7: CO2-Abscheideeinheit
- 8: zweite Leitung
- 9: weiterer erster Pufferspeicher
- 10: Rohmethanolleitung
- 11: Methanoldestillation
- 12: zweiter Pufferspeicher
- 13: Energiespeicher
- 14: Strom-zu-Wärme-Umwandler
- 15: Speicher für thermische Energie
- 16: Stellglied
- 17: Steuereinrichtung
- 18: Steuerbefehl
- 19: Echtzeit-Daten
- 20: Simulations-/Vorhersagemodell
- 21: Prognosedaten
- 22: Reines Methanol

## Patentansprüche

1. Verfahren zur Herstellung von Methanol, bei dem
a) ein erster Eduktstrom mit einem ersten Edukt in Form von Wasserstoff und ein zweiter Eduktstrom mit einem zweiten Edukt in Form eines kohlenstoffoxidhaltigen Gases bereitgestellt werden;
b) das erste und das zweite Edukt in eine Synthesereaktoranordnung (3) eingespeist werden; und
c) die Methanolsynthese in der Synthesereaktoranordnung (3) durchgeführt wird, wodurch ein Produktstrom, enthaltend Methanol, gebildet wird;
wobei das Verfahren ferner vorsieht:
die Bereitstellung wenigstens eines ersten Pufferspeichers (5, 9) zur Zwischenspeicherung wenigstens eines der Edukte vor der Synthesereaktoranordnung (3); das Ermitteln der Zuflussrate des wenigstens einen Edukts in die Synthesereaktoranordnung (3); und das Erstellen einer Prognose hinsichtlich einer Änderungsrate der Zuflussrate auf der Grundlage von Einflussgrößen, von denen die Zuflussrate des wenigstens einen Edukts abhängig ist;
wobei,
wenn die Prognose eine positive Änderungsrate anzeigt, deren Betrag einen vordefinierten ersten Schwellenwert überschreitet, zumindest ein Teil des Stroms des wenigstens einen Edukts in den ersten Pufferspeicher (5, 9) umgeleitet wird; und/oder
wenn die Prognose eine negative Änderungsrate anzeigt, deren Betrag einen vordefinierten zweiten Schwellenwert überschreitet, das wenigstens eine Edukt aus dem ersten Pufferspeicher (5, 9) in die Synthesereaktoranordnung (3) eingespeist wird,
um die Änderungsrate auf einen vordefinierten Wert oder vordefinierten Bereich einzustellen.

2. Verfahren nach Anspruch 1, wobei das erste und das zweite Edukt in getrennten Eduktströmen bereitgestellt werden, wobei wenigstens für einen der Eduktströme ein separater Pufferspeicher (5, 9) bereitgestellt wird.

3. Verfahren nach Anspruch 1 oder 2, wobei der erste und zweite Eduktstrom in separaten ersten Pufferspeichen zwischengespeichert werden.

4. Verfahren nach einem der vorhergehenden Ansprüche, ferner umfassend:
d) die Zuführung des in der Synthesereaktoranordnung (3) produzierten Produktstroms in eine Destillation (11);
die Bereitstellung eines zweiten Pufferspeichers (12) zur Zwischenspeicherung des Produktstroms; und
das Ermitteln der Zuflussrate des Produktstroms in die Destillation (11);
wobei der Produktstrom zumindest teilweise in den zweiten Pufferspeicher (12) umgeleitet wird oder ein Produktstrom aus dem zweiten Pufferspeicher (12) in die Destillation eingespeist wird, um Änderungen der Zuflussrate des Produktstroms von der Synthesereaktoranordnung (3) auszugleichen.

5. Verfahren nach Anspruch 4, ferner umfassend
das Erstellen einer Prognose hinsichtlich einer Änderungsrate des Produktstroms in die Destillation (11) auf der Grundlage von Einflussgrößen, von denen der die Zuflussrate des Produktstroms abhängig ist,
wobei
wenn die Prognose eine positive Änderungsrate anzeigt, deren Betrag einen vordefinierten dritten Schwellenwert überschreitet, zumindest ein Teil des Produktstroms in den zweiten Pufferspeicher (12) umgeleitet; und/oder
wenn die Prognose eine negative Änderungsrate anzeigt, deren Betrag einen vordefinierten vierten Schwellenwert überschreitet, ein Produktstrom aus dem Pufferspeicher (12) in die Destillation (11) eingespeist wird,
um die Änderungsrate der Zuflussrate des Produktstroms in die Destillation (11) auf einen vordefinierten Wert oder vordefinierten Bereich einzustellen.

6. Verfahren nach einem der vorhergehenden Ansprüche, wobei das zweite Edukt Kohlenstoffdioxid und/oder Kohlenstoffmonoxid und/oder ein Synthesegasgemisch, welches zumindest Wasserstoff und Kohlenmonoxid umfasst, ist.

7. Verfahren nach einem der vorhergehenden Ansprüche, wobei der Wasserstoff durch Wasserelektrolyse, vorzugsweise zumindest teilweise mit erneuerbarer Energie, bereitgestellt wird.

8. Verfahren nach Anspruch 7, ferner umfassend das Erstellen einer Prognose hinsichtlich der Energiebereitstellung für die Wasserelektrolyse auf Grundlage von Parametern, die die Bereitstellung von Energie für die Wasserelektrolyse beeinflussen.

9. Verfahren nach Anspruch 8, wobei das Verfahren ferner vorsieht:
a) einen Ramp-up-Betrieb, bei dem ein prognostizierter Anstieg einer Energiebereitstellung für die Wasserelektrolyse zu einer Erhöhung der Zuflussrate des ersten Edukts führt, wobei das erste Edukt zumindest teilweise in dem ersten Pufferspeicher (5) derart zwischengespeichert wird, dass die Änderungsrate der Zuflussrate des in die Synthesereaktoranordnung (3) eingespeisten ersten Edukts auf eine vordefinierte Rate begrenzt wird;
und/oder
b) einen Ramp-down-Betrieb, bei dem ein prognostizierter Rückgang einer Energieversorgung für die Wasserelektrolyse zu einer Verringerung der Zuflussrate des ersten Edukts führt, wobei das erste Edukt aus dem ersten Pufferspeicher (5) in die Synthesereaktoranordnung (3) derart eingespeist wird, dass die Änderungsrate der Zuflussrate des in die Synthesereaktoranordnung (3) eingespeisten ersten Edukts auf eine vorbestimmte Rate begrenzt wird.

10. Verfahren nach einem der vorhergehenden Ansprüche, wobei Wärme aus der Synthesereaktoranordnung (3) und/oder der Destillation (11) entnommen und zwischengespeichert und/oder durch Energiezufuhr erzeugt wird,
wobei das Verfahren ferner einen Standby-Betrieb umfasst,
wobei der Standby -Betrieb aufgenommen wird, wenn die prognostizierte Zuflussrate wenigstens eines der ersten und zweiten Edukte in die Synthesereaktoranordnung (3) unter einen vorbestimmten Schwellenwert sinkt,
wobei Duck- und Temperaturbedingungen in der Synthesereaktoranordnung (3) und/oder der Destillation (11) auf vorbestimmte Werte geregelt werden, wobei die durch Energiezufuhr erzeugte Wärme in die Synthesereaktoranordnung (3) und/oder die Destillation (11) geleitet wird.

11. Verfahren nach Anspruch 10, wobei der Standby-Betrieb wieder aufgehoben wird, wenn eine prognostizierte Zuflussrate von wenigstens einem der Eduktströme einen vorbestimmten Schwellenwert erreicht, wobei vorzugsweise die Einleitung der Wärme unterbrochen wird.

12. Verfahren nach einem der vorhergehenden Ansprüche, wobei
das erste Edukt in dem ersten Pufferspeicher (5) zwischengespeichert wird,
das zweite Edukt in dem ersten Pufferspeicher (9) zwischengespeichert wird; das erste und das zweite Edukt gemeinsam in dem ersten Pufferspeicher zwischengespeichert werden; oder
das erste Edukt und das zweite Edukt in separaten ersten Pufferspeichern (5, 9) zwischengespeichert werden.

13. Verfahren nach einem der vorhergehenden Ansprüche, wobei das Erstellen von Prognosen das Auswerten von Einflussgrößen, insbesondere Leistungsdaten von Betriebsparametern des Methanol-Herstellungsprozesses; und das Berechnen einer Wahrscheinlichkeit für das Auftreten einer Änderungsrate umfasst, wobei, wenn die Wahrscheinlichkeit einen vorbestimmten Schwellenwert überschreitet, die Änderungsrate als prognostiziert gilt.

14. Anlage zur Methanolherstellung, umfassend:
eine Synthesereaktoranordnung (3),
eine Destillation (11);
eine oder mehrere Quellen (2, 6) für ein erstes Eduktgas und ein zweites Eduktgas, wobei die eine oder die mehreren Quellen (2, 6) über eine gemeinsame oder über separate erste Leitungen (4, 8) mit der Synthesereaktoranordnung (3) verbunden sind,
wobei wenigstens eine der Leitungen (4, 8) mit einem ersten Pufferspeicher (5, 9) verbunden ist; und
eine zweite Leitung (10), die die Synthesereaktoranordnung (3) mit der Destillation (11) verbindet, wobei die zweite Leitung (10) einem zweiten Pufferspeicher (12) verbunden ist;
eine Steuereinrichtung (17), die dazu eingerichtet ist, die Anordnung mit einem Verfahren nach einem der Ansprüche 1 bis 13 zu betreiben.
